# EUROPEAN PATENT SPECIFICATION

(11) **EP 4 585 171 B1**
(45) Date of publication and mention of the grant of the patent: **09.12.2026**
(21) Application number: 25174496.7
(22) Date of filing: 23.09.2019
(51) Int. Cl.: A61B 17/32, A61B 17/22, A61B 18/00

(54) **ULTRASONIC TIP WITH PROTRUSION DEFINING A PREASPIRATION HOLE**
ULTRASCHALLSPITZE MIT EINEM EINE PRÄASPIRATIONSÖFFNUNG DEFINIERENDEN VORSPRUNG
POINTE ULTRASONORE AVEC SAILLIE DÉFINISSANT UN TROU DE PRÉASPIRATION

(30) Priority: 24.09.2018 US 201862735440 P
(43) Date of publication of application: 16.07.2025
(62) Document number(s) of the earlier application(s) in accordance with Art. 76 EPC: 19783785.9 / 3 856 051
(73) Proprietor: Stryker European Operations Holdings LLC, Portage, MI 49002-9711 (US)
(72) Inventor: GRAS, Guillaume, 3053 Muenchenbuchsee (CH); HEAVEY, Cathal, Midleton, Co. Cork, P25VW86 (IE); MC CAETHY, Conor, Cork (IE)
(74) Representative: Röthinger, Rainer

(56) References cited:
- WO-A1-95/24865
- US-A- 4 516 398
- US-A- 5 163 433
- US-A1- 2006 235 306
- US-A1- 2018 200 109
- US-A1- 2018 250 031
- US-B2- 6 955 680
- US-B2- 8 512 340

## Description

### RELATED APPLICATIONS

This patent application claims priority to and all the benefits of U.S. Provisional Patent Application No. 62/735,440 filed on September 24, 2018.

### BACKGROUND

As medical professionals strive to reduce the size of the incisions and the amount of recovery time required following surgical procedures, the sizes of medical instruments used in such procedures have become smaller. Medical instruments utilized in performing such surgical procedures may include the use of a cutting accessory, such as an ultrasonic tip. In performing a cutting, shaving, or shaping operation, the cutting accessory will be exposed to varying amounts of force, creating stresses within the cutting accessory. The cutting accessories may also be used in coordination with irrigation or aspiration, that is, suction, to reduce heat and/or remove debris at the surgical site. Irrigation may also be utilized as a cutting medium.

According to US 5,163,433, an ultrasound type treatment apparatus includes an inner sheath covering an ultrasonic transmission member having a first suction passage and defining a space relative to the ultrasound transmission member and an outer sheath covering the inner sheath and defining a space relative to the inner sheath. In the treatment apparatus, a liquid medium supply passage is provided at one of the space between the ultrasound transmission member and the inner sheath and space between the inner sheet and the outer sheet to supply a liquid medium to the distal end of the ultrasound transmission member. A second suction passage is formed at the other of the space between the ultrasound transmission means and the inner sheath and space between the inner sheath and the outer sheath. The first suction passage is connected by a suction tube to the second suction passage.

### SUMMARY

An ultrasonic sleeve assembly according to the present invention is defined in claim 1. Optional features are defined in the dependent claims.

### BRIEF DESCRIPTION OF THE DRAWINGS

Advantages of the present disclosure will be readily appreciated as the same becomes better understood by reference to the following detailed description when considered in connection with the accompanying drawings.
Figure 1 is a top view of an ultrasonic surgical handpiece assembly.
Figure 2 is a sectional view of the ultrasonic surgical handpiece assembly of Figure 1 in the direction of section line arrows 2.
Figure 3 is a top, angled view of an ultrasonic tip and an irrigation sleeve with the ultrasonic tip positioned within the irrigation sleeve.
Figure 4 is sectional view of the ultrasonic tip and the irrigation sleeve of Figure 3 in the direction of section line arrows 4.
Figure 5 is a perspective view of a distal portion of the ultrasonic tip including a protrusion and a cutting feature.
Figure 6A is a side view of a first configuration of an ultrasonic tip including the protrusion and cutting feature.
Figure 6B is a cross-sectional view of the ultrasonic tip along the 6B axis of Figure 6A in the direction of section line arrows 6B.
Figure 6C is a cross-sectional view of the ultrasonic tip along the 6C axis of Figure 6A in the direction of section line arrows 6C.
Figure 6D is a cross-sectional view of the ultrasonic tip along the 6D axis shown in Figure 6A in the direction of section line arrows 6D.
Figure 6E is a cross-sectional view of the ultrasonic tip along the 6E axis shown in Figure 6A in the direction of section line arrows 6E.
Figure 6F is a cross-sectional view of the ultrasonic tip along the 6F axis shown in Figure 6A in the direction of section line arrows 6F.
Figure 7 is a sectional view of the ultrasonic tip of Figure 6A in the direction of section line arrows 7.
Figure 8 is a sectional view of the distal portion of the ultrasonic tip of Figure 5, including the protrusion and the cutting feature in the direction of section line arrows 8.
Figure 9 is an enlarged sectional view of a distal portion of ultrasonic tip and the irrigation sleeve of a portion of Figure 4 in oval 9.
Figure 10A is a bottom view of a first configuration of an ultrasonic tip including the protrusion and cutting feature.
Figure 10B is a perspective view of the first configuration of a cutting feature at the distal region of the ultrasonic tip of Figure 10A.
Figure 10C is a perspective view of a second configuration of a cutting feature at the distal region of the ultrasonic tip of Figure 10A.
Figure 10D is a perspective view of a third configuration of a cutting feature at the distal region of the ultrasonic tip of Figure 10A.
Figure 10E is a perspective view of a fourth configuration of a cutting feature at the distal region of the ultrasonic tip of Figure 10A.

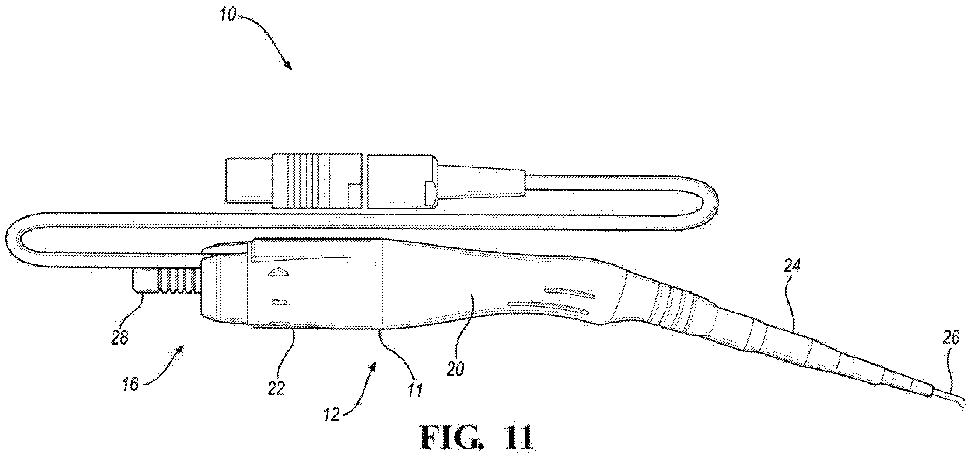

Figure 11 is a side view of the ultrasonic surgical handpiece assembly of Figure 1.

### DETAILED DESCRIPTION

One example of a surgical instrument that may utilize irrigation and/or aspiration systems is an ultrasonic surgical handpiece. Generally, one or more lines may be coupled to the ultrasonic surgical handpiece to supply irrigation and/or suction. The ultrasonic surgical handpiece may further comprise a sleeve comprising one or more lumens that may be utilized to direct fluid from an irrigation source toward the surgical site and/or the cutting accessory, i.e., the ultrasonic tip.

An exemplary ultrasonic tip is for use with a surgical handpiece to produce both longitudinal and torsional motion, the handpiece including an ultrasonic transducer disposed within a housing. The ultrasonic tip comprises a shaft, a cutting feature, and a protrusion. The shaft has a longitudinal axis extending between a proximal end and a distal end of the shaft. The proximal end has a first diameter. The distal end has a second diameter. The first diameter is greater than the second diameter. The shaft comprises a vibration conversion mechanism for converting a vibration energy transmitted from the ultrasonic transducer into a composite vibration composed of a longitudinal vibration along the longitudinal axis and a torsional vibration. The cutting feature is coupled to the distal end of the shaft. An aspiration lumen is defined by the shaft. The aspiration lumen is configured to extend along the longitudinal axis of the shaft. The aspiration lumen is configured to be placed in fluid communication with the handpiece. The protrusion is on the shaft and is positioned between the distal end and the proximal end. The protrusion is positioned distal to the vibration conversion mechanism along the longitudinal axis. A portion of the protrusion has a third diameter. The third diameter is less than the first diameter and the third diameter is greater than the second diameter. An aperture is defined by the protrusion. The aperture is in fluid communication with the aspiration lumen. The protrusion reinforces an area surrounding the aperture.

The proximal end of the shaft may comprise a coupling feature configured to removeably couple the shaft to the transducer of the handpiece.

The coupling feature may comprise a plurality of threads configured to couple to the transducer of the handpiece.

The vibration conversion mechanism may comprise one or more helical groove portions formed on an external surface of the shaft.

An axis of the aperture may be transverse to the longitudinal axis of the ultrasonic tip.

The cutting feature may comprise a cutting face facing in a direction at an angle equal to or less than 90 degrees to the longitudinal axis.

The cutting feature may have a cutting face arranged radially outward from the longitudinal axis. The cutting face may comprise one or more teeth.

The protrusion may comprise a first taper region, a median region, and a second taper region. An external surface of the median region may be generally parallel to the longitudinal axis. The median region may be disposed between the first taper region and the second taper region. The first taper region may be proximal to the median region and have a positive slope. The second taper region may be distal to the median region and have a negative slope.

The aspiration lumen may be open at the distal end and at the proximal end of the shaft.

An exemplary ultrasonic sleeve assembly is for use with a surgical handpiece comprising an ultrasonic transducer. The ultrasonic sleeve assembly comprises a cutting tip and an irrigation sleeve. The cutting tip comprises a shaft, a vibration conversion mechanism, a cutting feature and a protrusion. The shaft extends between a proximal end and a distal end. The proximal end comprises a coupling feature configured to removeably couple the shaft to the handpiece. The proximal end comprises a first diameter and the distal end comprises a second diameter. The first diameter is greater than the second diameter. The vibration conversion mechanism is for converting a vibration energy transmitted from the ultrasonic transducer into a composite vibration composed of a longitudinal vibration along a longitudinal axis and a torsional vibration. The cutting feature is coupled to the distal end of the shaft. An aspiration lumen is defined by the shaft and is configured to extend along the longitudinal axis of the shaft. The aspiration lumen is configured to be in fluid communication with the surgical handpiece when the shaft is coupled to the surgical handpiece. The protrusion is on the shaft and is positioned distal of the vibration conversion mechanism. A portion of the protrusion has a third diameter. The third diameter is less than the first diameter. The third diameter is greater than the second diameter. The protrusion is configured to strengthen the shaft. An aperture is defined by the protrusion and is in fluid communication with the aspiration lumen. The irrigation sleeve is configured to surround a portion of the shaft when the irrigation sleeve and the cutting tip are coupled to the handpiece. The irrigation sleeve defines a lumen comprising a proximal end and a distal end. The proximal end of the lumen comprises a coupling mechanism configured to removeably couple to the handpiece. The irrigation sleeve further defines an irrigation channel separate from the lumen. The irrigation channel has a distal end and a proximal end. The proximal end of the irrigation channel is configured to releasably couple to an irrigation source. The irrigation channel is configured to carry fluid to the lumen through an irrigation port. The irrigation port is in fluid communication with the irrigation channel and the lumen.

The coupling feature may comprise a plurality of threads configured to couple to the transducer of the handpiece.

The vibration conversion mechanism may comprise one or more helical groove portions formed on an external surface of the shaft.

The cutting feature may comprise a cutting face facing in a direction perpendicular to the longitudinal axis.

The aperture may be positioned in a radially opposed direction from the cutting face.

The cutting feature may have a cutting face arranged radially outward from the longitudinal axis. The cutting face may comprise one or more teeth.

The protrusion may comprise a first taper region, a median region, and a second taper region. An external surface of the median region may be generally parallel to the longitudinal axis. The median region may be disposed between the first taper region and the second taper region. The first taper region may be proximal to the median region and may have a positive slope. The second taper region may be distal to the median region and may have a negative slope.

The aspiration lumen may be open at the distal end and the proximal end of the shaft.

An exemplary ultrasonic cutting system for producing both longitudinal and torsional motion comprises a handpiece, a cutting tip and an irrigation sleeve. The handpiece comprises a transducer disposed within a housing. The cutting tip is coupled to the handpiece and comprises a shaft and a cutting feature. The shaft comprises a distal end and a proximal end. The proximal end comprises a coupling feature configured to removeably couple the shaft to the handpiece. The cutting feature is coupled to the distal end of the shaft. An aspiration lumen is defined by the shaft. The aspiration lumen is configured to extend along a longitudinal axis of the shaft. The aspiration lumen is in fluid communication with the handpiece. An aperture is in fluid communication with the aspiration lumen, and is located between the proximal end and the distal end of the shaft. The aperture has a proximal end and a distal end. An irrigation sleeve is coupled to the handpiece. The irrigation sleeve surrounds a portion of the shaft, and has a proximal end and a distal end. The irrigation sleeve defines a lumen. The proximal end of the irrigation sleeve has a coupling mechanism configured to removeably couple the irrigation sleeve to the handpiece. The irrigation sleeve further defines an irrigation channel comprising a distal end and a proximal end. The proximal end of the irrigation channel is configured to receive irrigation fluid from an irrigation source. An irrigation port is in fluid communication with the distal end of the irrigation channel. The irrigation port is configured to dispense irrigation fluid toward the shaft. The distal end of the irrigation sleeve is positioned proximal to the distal end of the shaft. The irrigation port is positioned proximal relative to the aperture along the longitudinal axis. The distal end of the irrigation sleeve is distal to the distal end of the aperture such that the irrigation sleeve encompasses an entirety of the aperture.

The distal end of the irrigation sleeve may extend beyond the distal end of the aperture in a distal direction along the longitudinal axis.

The coupling feature may comprise a plurality of threads configured to couple to the transducer of the handpiece.

The shaft may comprise a vibration conversion mechanism for converting a vibration energy transmitted from the transducer into a composite vibration composed of a longitudinal vibration along the longitudinal axis and a torsional vibration.

The vibration conversion mechanism may comprise one or more helical groove portions formed on an external surface of the shaft.

The cutting feature may comprise a cutting face facing in a direction perpendicular to the longitudinal axis.

The aperture may be positioned in a radially opposed direction from the cutting face.

The cutting feature may have a cutting face arranged radially outward from the longitudinal axis and wherein the cutting face comprises one or more teeth.

The aspiration lumen may be open at the distal end and the proximal end of the shaft.

An exemplary ultrasonic tip is for use with a surgical handpiece to produce both longitudinal and torsional motion, the surgical handpiece including an ultrasonic transducer. The ultrasonic tip comprises a shaft, a cutting feature and a protrusion. The shaft extends between a proximal end and a distal end. The proximal end has a first cross-sectional area and a second cross-sectional area. The first cross-sectional area is greater than the second cross-sectional area. The shaft comprises a vibration conversion mechanism for converting a vibration energy transmitted from the ultrasonic transducer into a composite vibration composed of a longitudinal vibration along a longitudinal axis and a torsional vibration. The cutting feature is coupled to the distal end of the shaft. An aspiration lumen is defined by the shaft. The aspiration lumen is configured to extend along the longitudinal axis of the shaft. The aspiration lumen is configured to be placed in fluid communication with the handpiece. The protrusion is on the shaft and is positioned between the distal end and the proximal end. The protrusion is positioned distal to the vibration conversion mechanism along the longitudinal axis. A portion of the protrusion has a third cross-sectional area. The third cross-sectional area is less than the first cross-sectional area. The third cross-sectional area is greater than the second cross-sectional area. An aperture is defined by the protrusion and is in fluid communication with the aspiration lumen. The protrusion reinforces an area surrounding the aperture.

An exemplary ultrasonic tip is for use with a surgical handpiece that includes an ultrasonic transducer disposed within a housing. The ultrasonic tip comprises a shaft, a cutting feature and a protrusion. The shaft comprises a distal region, an intermediate region and a proximal region. The intermediate region has a first diameter. The distal region has a second diameter. The first diameter is greater than the second diameter. The shaft comprises a vibration conversion mechanism for converting a vibration energy transmitted from the ultrasonic transducer into a composite vibration composed of a longitudinal vibration along a longitudinal axis and a torsional vibration. The cutting feature is coupled to the distal region of the shaft. A first lumen is defined by the shaft. The first lumen is configured to extend along the longitudinal axis of the shaft. The first lumen is configured to be placed in fluid communication with the handpiece. The protrusion is on the shaft and is positioned between the intermediate region and the distal region. The intermediate region is positioned between the protrusion and the vibration conversion mechanism. A portion of the protrusion has a third diameter. The third diameter is less than the first diameter, and the third diameter is greater than the second diameter. An aperture is defined by the protrusion The aperture is in fluid communication with the first lumen. The protrusion reinforces an area surrounding the aperture.

An exemplary ultrasonic tip is for use with a surgical handpiece to produce both longitudinal and torsional motion, the handpiece including an ultrasonic transducer. The ultrasonic tip comprises a shaft, a cutting feature and a protrusion. The shaft extends between a proximal end and a distal end. The proximal end has a first cross-sectional area. The distal end has a second cross-sectional area. The first cross-sectional area is greater than the second cross-sectional area. The cutting feature is coupled to the distal end of the shaft. An aspiration lumen is defined by the shaft. The aspiration lumen is configured to extend along a longitudinal axis of the shaft. The aspiration lumen is configured to be placed in fluid communication with the handpiece. The protrusion is on the shaft and is positioned between the distal end and the proximal end. A portion of the protrusion has a third cross-sectional area. The third cross-sectional area is less than the first cross-sectional area. The third cross-sectional area is greater than the second cross-sectional area. An aperture is defined by the protrusion and in fluid communication with the aspiration lumen. The protrusion reinforces an area surrounding the aperture.

An exemplary ultrasonic tip is for use with a surgical handpiece to produce both longitudinal and torsional motion, the handpiece including an ultrasonic transducer disposed within a housing. The ultrasonic tip comprises a shaft, a cutting feature and a protrusion. The shaft has a longitudinal axis extending between a proximal end and a distal end. The proximal end has a first diameter and the distal end has a second diameter. The first diameter is greater than the second diameter. The cutting feature is coupled to the distal end of the shaft. An aspiration lumen is defined by the shaft. The aspiration lumen is configured to extend along the longitudinal axis of the shaft. The aspiration lumen is configured to be placed in fluid communication with the handpiece. The protrusion is on the shaft and is positioned between the distal end and the proximal end. A portion of the protrusion has a third diameter. The third diameter is less than the first diameter. The third diameter is greater than the second diameter. An aperture is defined by the protrusion and is in fluid communication with the aspiration lumen. The protrusion reinforces an area surrounding the aperture.

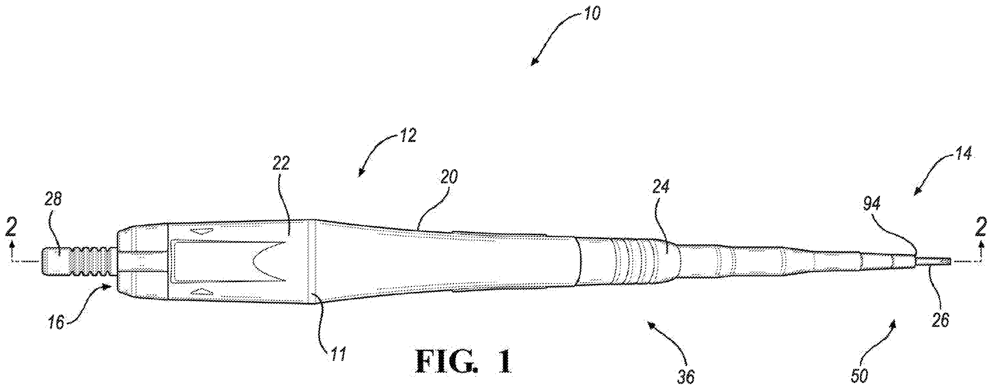

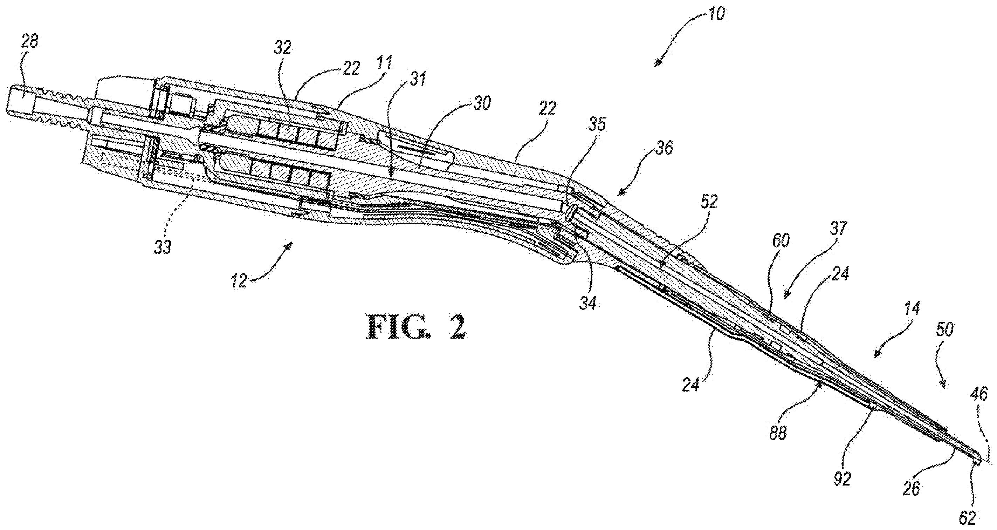

Figures 1 and 2 illustrate an exemplary configuration of an ultrasonic surgical handpiece assembly 10, which may comprise part of an ultrasonic cutting system 12, that may be utilized by a medical professional to remove biological material from a patient. The ultrasonic surgical handpiece assembly 10 may comprise an ultrasonic handpiece 11 including a proximal housing portion 22 and a distal housing portion 20. An irrigation sleeve 24 may be removably coupled to the distal housing portion 20 of the ultrasonic surgical handpiece 11. The irrigation sleeve 24 and an ultrasonic tip 26, alternatively identified herein as a cutting tip 26, may be configured such that the irrigation sleeve 24 surrounds at least a portion of the ultrasonic tip 26 along a length of the ultrasonic tip 26 when both the irrigation sleeve 24 and the ultrasonic tip 26 are coupled to the ultrasonic handpiece 11. The irrigation sleeve 24 and the ultrasonic tip 26 may at least in part comprise an ultrasonic sleeve assembly 14.

Figure 2 illustrates a sectional view of the ultrasonic surgical handpiece assembly 10 of Figure 1. As illustrated in Figure 2, the ultrasonic handpiece 11 may comprise a transducer 32, which may be an ultrasonic transducer 32, disposed within a void defined by the distal housing portion 20 and the proximal housing portion 22 of the ultrasonic handpiece 11. The transducer 32 may comprise a piezoelectric element or a magnetostrictive element configured to generate mechanical energy.

The ultrasonic handpiece 11 may also comprise a horn 30 that may be at least partially disposed within the void defined by the distal housing portion 20 and the proximal housing portion 22 of the ultrasonic handpiece 11. The horn 30 may comprise a distal end and a proximal end. The proximal end of the horn 30 may be coupled to a distal end of the transducer 32. The transducer 32 may be configured to provide the mechanical energy generated by the piezoelectric element or magnetostrictive element to the horn 30. The horn 30 may also be configured to define a horn lumen 31 that extends from the distal end to the proximal end of the horn 30 for fluid connection to a nipple 28 at a proximal end 16 of the proximal housing portion 22. The nipple 28 may be used to connect the handpiece 11 to a vacuum source (not shown). The horn lumen 31 may define a portion of a passageway that extends through the ultrasonic handpiece 11 to provide aspiration to the ultrasonic tip 26.

The ultrasonic handpiece 11 may further comprise an irrigation line 33 that is disposed within the void defined by the distal housing portion 20 and the proximal housing portion 22 of the ultrasonic handpiece 11. The irrigation line 33 may be configured to extend from the proximal end 16 to the distal end of the ultrasonic handpiece 11. The irrigation line 33 may serve to channel water from an irrigation system that is coupled to the ultrasonic handpiece 11 through the ultrasonic handpiece 11 to the irrigation sleeve 24. It should be appreciated that the irrigation line 33 may route directly from an irrigation source (not shown) to the ultrasonic sleeve (i.e., the irrigation line need not be always routed through the handpiece).

The ultrasonic tip 26 may comprise a shaft 48 that comprises a distal region 50, alternatively referenced to herein as a distal end 50, an intermediate region 37, and a proximal region 36, alternatively reference to herein as a proximal region 36. The shaft also has a distal end and a proximal end, with the proximal region being adjacent the proximal end and the distal region being adjacent the distal end. The ultrasonic tip may also comprise a coupling feature 34 positioned at the proximal region 36 of the shaft 48 and is configured to couple the proximal region 36 of the ultrasonic tip 26 to the distal end of the horn 30 to allow the horn 30 to be in mechanical communication with the ultrasonic tip 26. The coupling feature 34 may be a threaded coupler configured to engage a corresponding threaded coupler 35 on the distal end of the horn 30. The ultrasonic tip 26 may be threaded into the horn 30 and tightened to a predetermined torque specification to removably secure the ultrasonic tip 26 to the ultrasonic handpiece 11. While not illustrated in the figures, it is contemplated that the coupling feature 34 may be configured as a quick connection, quarter turn fitting, or similar coupling mechanism. It is further contemplated that the coupling feature 34 may be configured to permanently affix the ultrasonic tip 26 to the handpiece 11. For example, the ultrasonic tip 26 may be coupled to the ultrasonic handpiece 11 by a weld, epoxy, or similar coupling method. Alternatively, it is also contemplated that the ultrasonic tip 26 and the horn 30 may be formed as a unitary component.

The shaft 48 of the ultrasonic tip 26 may also be configured to define an aspiration lumen 52 that extends from the proximal region 36, through the intermediate region 37, to the distal region 50 of the ultrasonic tip 26. The aspiration lumen 52 may be oriented to be generally parallel to and may extend along a longitudinal axis. The aspiration lumen 52 of the ultrasonic tip 26 may be configured to form a fluid passageway with the lumen 31 of the horn 30 when the ultrasonic tip 26 is coupled to the horn 30. Otherwise stated, the aspiration lumen 52 may be configured to be placed in fluid communication with the handpiece 11, and the lumen 31 of the horn 30 in particular. The aspiration lumen 52 is open at the distal region 50 of the shaft 48. The aspiration lumen 52 of the ultrasonic tip 26 may be configured to provide aspiration away from the surgical site. For example, the aspiration lumen 52 may be used to vacuum fluid and biological tissue away from the distal end 50 of the ultrasonic tip 26. The aspiration lumen 52 may be in fluid communication with a lumen defined by the cutting feature 62.

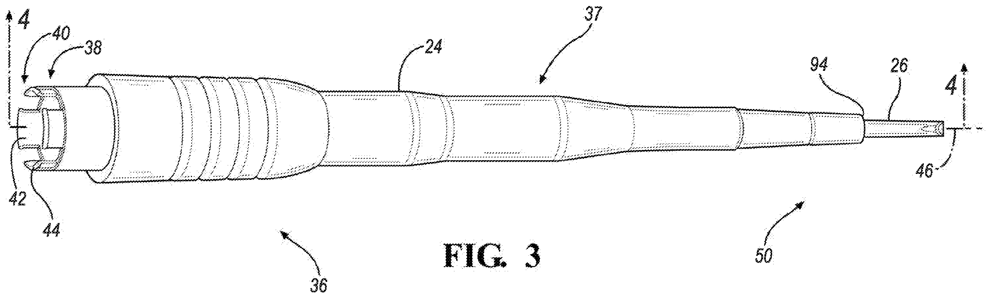

Figure 3 illustrates a top view of an exemplary configuration of the irrigation sleeve 24 having a distal end 94 and a proximal end. The irrigation sleeve 24 may comprise an irrigation sleeve coupling mechanism 38 on the proximal end of the irrigation sleeve 24. The irrigation sleeve coupling mechanism 38 may comprise one or more fingers 42 extending proximally from the proximal end of the irrigation sleeve 24. Each of the one or more fingers 42 may comprise a tab 44 extending in a radially outward direction relative to the longitudinal axis 46 of the irrigation sleeve 24. The fingers 42 act as the male fitting configured to couple with a female fitting (not shown) on the handpiece 11 to create a snap-fit 40 or interference fit. It is contemplated that other types of irrigation sleeve coupling mechanisms 38 may be used to couple the irrigation sleeve 24 to the ultrasonic handpiece 11. For example, the irrigation sleeve coupling mechanisms 38 may be configured as a threaded connection.

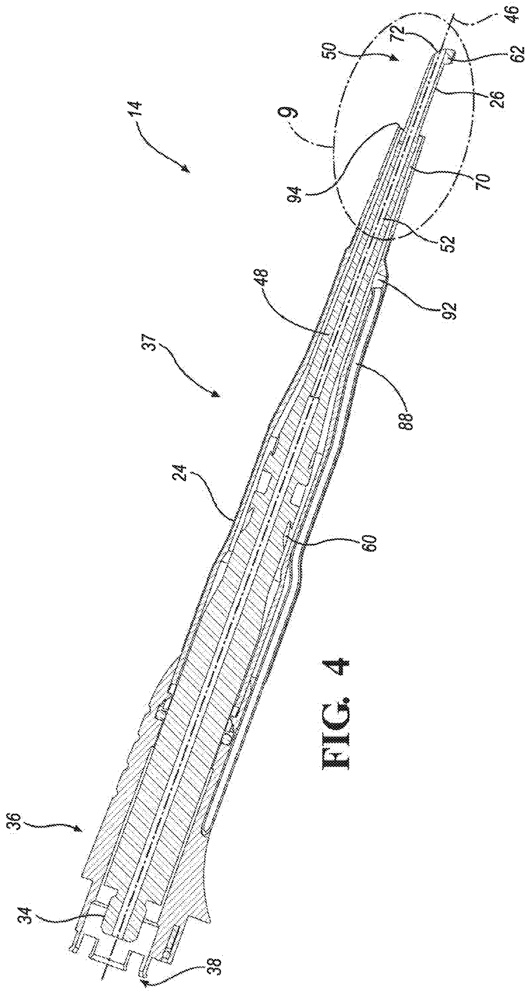

Referring to Figure 4, a sectional view of the ultrasonic tip 26 at least partially disposed within a lumen 70 defined by the irrigation sleeve 24 is illustrated. The irrigation sleeve 24 may comprise an irrigation channel 88 that has a proximal end and a distal end. The proximal end of the irrigation channel 88 may be configured to couple to the distal end of the irrigation line 33 of the ultrasonic handpiece 11 or irrigation source. The irrigation channel 88 may be configured to run adjacent to the lumen 70 defined by the irrigation sleeve 24 and terminating at a port 92 disposed on the surface of the lumen 70 to provide irrigation fluid from the ultrasonic handpiece 11 to the ultrasonic tip 26 and the surgical site. The position of the port 92 may vary. For example, the port 92 may be adjacent the proximal end of the sleeve 24. Alternatively, the position of the port may be adjacent the distal end of the sleeve.

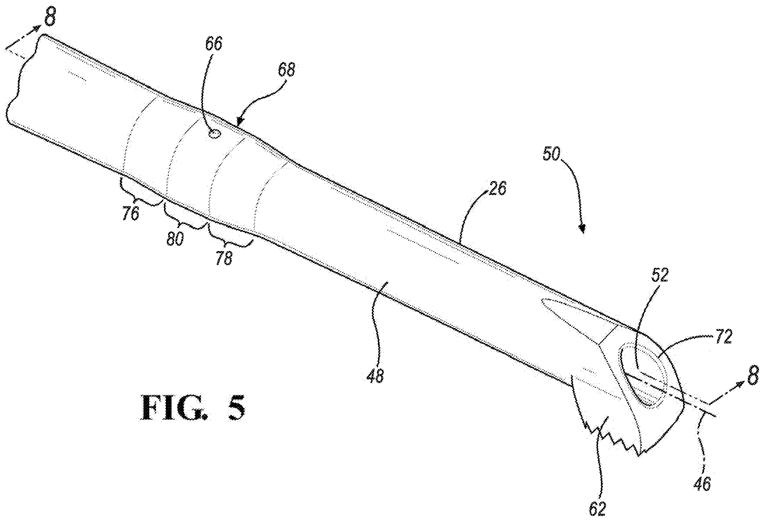

Referring to Figure 5, a portion of the distal region of the ultrasonic tip 26 is illustrated. The shaft 48 of the ultrasonic tip 26 may comprise a protrusion 68 at an intermediate point on the shaft 48 between the proximal region 36 and the distal region 50 of the ultrasonic tip 26. The protrusion 68 may be configured to extend radially about the shaft 48 of the ultrasonic tip 26. The protrusion 68 may comprise a first taper region 76, a median region 80, and a second taper region 78. The median region 80 is positioned between the first taper region 76 and the second taper region 78. In an exemplary configuration, the first taper region 76 may have a positive slope on the proximal side of the protrusion 68, and the second taper region 78 may have a negative slope on the distal side of said protrusion 68 relative to the longitudinal axis of the lumen of the shaft. The median region 80 may have no slope and may be parallel with respect to the longitudinal axis 46. In another configuration, the protrusion 68 may comprise a generally rounded or hemispherical geometry at the transition from the first taper region 76 to the median region 80 and then the second taper region 78. The diameter and cross-sectional area of the first taper region 76 and the second taper region 78 is less than the diameter and cross-sectional area of the median region 80. In another configuration, the protrusion 68 may comprise other multifaceted shapes, not shown. The protrusion 68 may be configured to have a progressive transition from the first taper region 76 to the median region 80 and then from the median region 80 to the second taper region 78. The progressive transition of the protrusion 68 may reduce the likelihood of creating an additional stress point in the shaft 48 that may have a higher probability of failure during ultrasonic vibration. Additionally, the progressive transition prevents disturbances of the fluid flow patterns about the ultrasonic tip 26 as the fluid moves distally 50 relative to the ultrasonic tip 26 from between the irrigation sleeve 24 and the ultrasonic tip 26 from the irrigation port 92. The progressive transition also allows for the ultrasonic tip 26 to be used at high power without experiencing high stress to the point that could cause breakage of the ultrasonic tip 26. The progressive transition may also improve the properties of the shaft 48 to allow the ultrasonic tip 26 to be operated at optimal frequencies.

The shaft 48 may be free from any abrupt steps from the length associated with diameter D4 to the length associated with diameter D5 and/or the length associated with diameter D3 to the length associated with D5. Free from any abrupt steps should mean that the angle of the slope of each of the transition regions is lower than 10, or 5 degrees relative to the external surface of the shaft 48.

The ultrasonic tip 26 may further comprise the aperture 66 in the shaft 48. The protrusion 68 defines the aperture 66, typically in the median region 80 of the protrusion 68. The aperture 66 is in fluid communication with the aspiration lumen 52. The axis of the aperture 66 may be transverse to the longitudinal axis 46 of the ultrasonic tip 26. In one configuration, the axis of the aperture 66 may be perpendicular to the longitudinal axis 46. The diameter of the aperture 66 is smaller than the diameter of the aspiration lumen 52.

The protrusion 68 reinforces the area of the shaft 48 near the aperture 66. Because the aperture 66 otherwise weakens the strength of the shaft 48 by the aperture 66, a thicker third diameter D3 of the shaft 48 at the protrusion 68 and may help to increase the structural integrity of the ultrasonic tip 26 when the ultrasonic tip 26 is subjected to ultrasonic movements in the torsional and longitudinal directions during use.

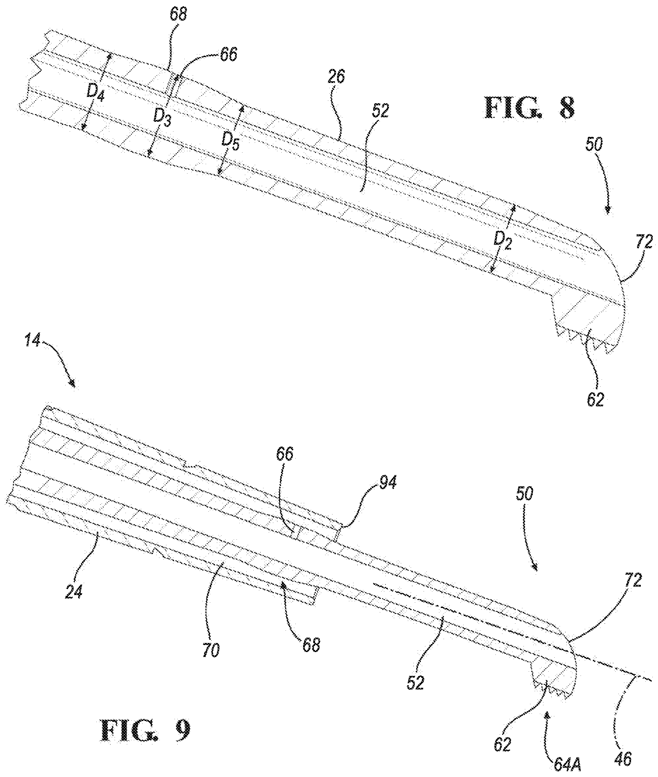

The cutting feature 62 and an aspiration lumen opening 72 may be positioned at the distal end of the shaft 48. Figures 8 and 9 illustrate the aspiration lumen opening 72 being open on the distal region 50 of the shaft 48 and/or cutting feature 62. The aspiration lumen opening 72 may be used to remove excess fluid and/or biological debris from the surgical site.

In certain configurations, the dimension of the protrusion 68 are configured based on the dimensions of the aperture 66. For example, the axial length of the median region 80 may be between 200-1000 % larger than the diameter of the aperture 66. In other configurations, the axial length of the median region 80 may be between 500-800% larger than the diameter of the aperture 66. Similarly, the length of the first and second tapered regions (76, 78) may also be related to the diameter of the aperture 66. For example, the combined axial length of the first tapered region 76, the median region 80, and the second tapered region 78 may be between 15000 and 24000 % larger than the diameter of the aperture 66. These dimensions ensure that the tip achieves the precise balance of stress reduction and cutting performance.

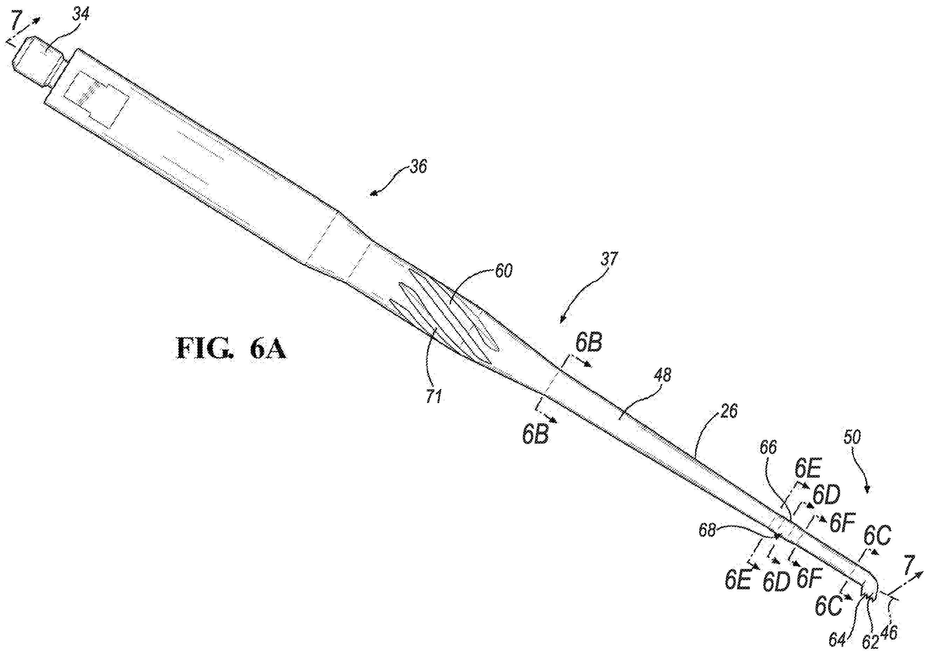

For example, the length of the protrusion 68 (i.e., the distance between the line at 6E and 6F in Figure 6A) may be approximately 2 mm, and could be between 1 mm and 6 mm in other configurations. The axial length (from the length at D4 to the length at D5) and radial thickness of the protrusion 68 may be related to the amount of material removed to create the aperture. For example, if the aperture 66 were to have a larger diameter, the diameter of the protrusion 68 and thickness would be greater. Conversely, if the aperture 66 were to have a smaller diameter, the thickness and length of the protrusion 68 would be less.

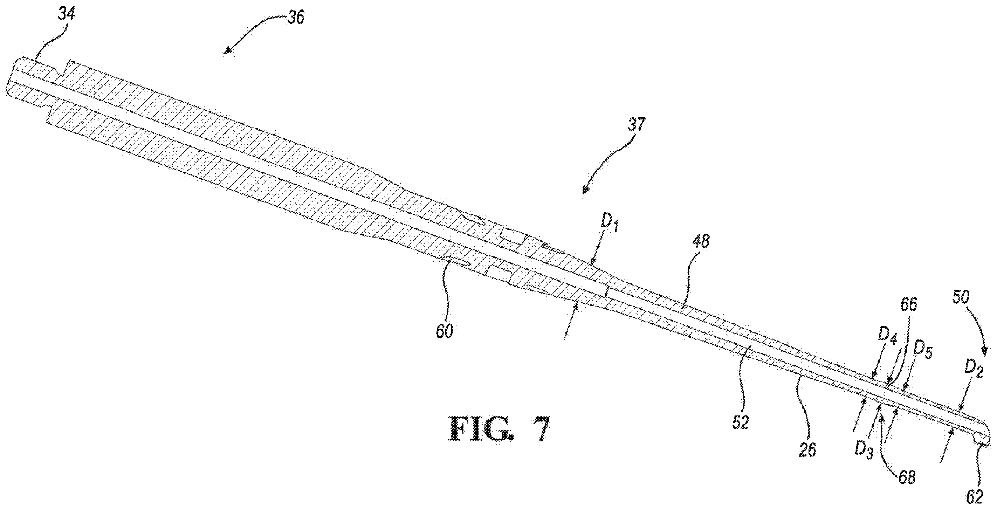

Figures 7 and 8 show the ultrasonic tip 26 comprising the shaft 48 and a protrusion 68 on the shaft 48 positioned between the distal region 50 and the proximal region 36. The protrusion 68 is positioned distal to the vibration conversion mechanism 60 along the longitudinal axis 46, as shown in Figure 6A. The shaft 48 at the protrusion 68 may have a third diameter D3 that is less than the first diameter D1 at the intermediate region 37 and greater than the second diameter D2 at the distal region 50.

As illustrated in Figures 6A-7, the diameter of the ultrasonic tip 26 varies from the proximal region 36, through the intermediate region 37, to the distal region 50 of the shaft 48. The shaft 48 comprises a first diameter D1 and comprises a second diameter D2, wherein the first diameter D1 is greater than the second diameter D2. The diameter D1 is positioned in the intermediate region 37 of the shaft 48 and the diameter D2 is positioned in the distal region 50 of the shaft.

The shaft 48 may be generally tapered along the longitudinal axis 46 from the proximal region 36, through the intermediate region 37, to the distal region 50. For example, the diameter of the shaft 48 may become smaller distally along the shaft 48 from the proximal region 36 to the distal region 50. One of a number of advantages provided by a tapered shaft 48, as it may reduce the size of the tip proximate and improve the user's line of sight while using the ultrasonic tip 26.

The shaft 48 may be made of a metal material such as titanium alloy, stainless steel, etc. or a non-metallic material such as a composite, depending on the application. In one example of the shaft 48, and ultrasonic tip 26 may be integral, unitary, and one-piece. In another example, the distal end 50 of the ultrasonic tip 26 may be attached to the shaft 48 by a suitable mechanism such as threads (not shown). It should be appreciated that metals are known in the art regarding high power ultrasonic components. It should also be appreciated that the diameters of the distal region 50 of the shaft 48 and the ultrasonic tip 26 have a relatively small diameter, for example less than one centimeter (1 cm), so as to work in a small opening of the patient. It should further be appreciated that the shaft 48 and the ultrasonic tip 26 may be scaled larger or smaller depending on the application.

Referring to Figure 6A, the ultrasonic tip 26 further comprises a vibration conversion mechanism 60 for converting a vibration energy transmitted from the ultrasonic transducer 32 into a composite vibration composed of a longitudinal vibration along a longitudinal axis 46 and a torsional vibration. The intermediate region 37 is distal the vibration conversion mechanism 60. The vibration conversion mechanism 60 may comprise one or more helical groove portions 71 on the surface of the shaft 48. These one or more groove portions 71 may be wound around a circumferential surface of the shaft 48, as shown. The vibration conversion mechanism 60 functions to convert the longitudinal vibration transmitted from the transducer 32 through the horn 30 into a composite vibration composed of a longitudinal vibration in the longitudinal axial 46 direction of the ultrasonic tip 26 and a torsional vibration having the longitudinal axis 46 of the ultrasonic tip 26 act as a fulcrum. It should be appreciated that the vibration conversion mechanism may alternatively take other forms suitable for converting a longitudinal motion into a composite motion. These alternative forms may be asymmetries in the cross-section shape of the shaft 48. Details regarding the vibration conversion mechanism may be found in U.S. Patent Nos. 6,497,715; 6,955,680; and 6,984,220.

Figure 6A also shows the ultrasonic tip 26 comprising the shaft 48 and a cutting feature 62 coupled to the distal region 50 of the shaft 48. The cutting feature 62 may be the part of the ultrasonic tip 26 that dissects or cuts a patient's biological tissue. A range of different cutting features 62 may be coupled to the distal end 50 of the shaft 48. In one example, the cutting feature 62 may have a cutting face 64 facing in a direction at an angle equal to or less than 90 degrees relative to the longitudinal axis 46. The terms cutting face and cutting surface may be used interchangeably herein. The cutting face 64 may be positioned in a manner that the center of the cutting face 64 is on an opposing side of the shaft 48 relative to the aperture 66. The cutting feature may be configured to cut with torsional motion.

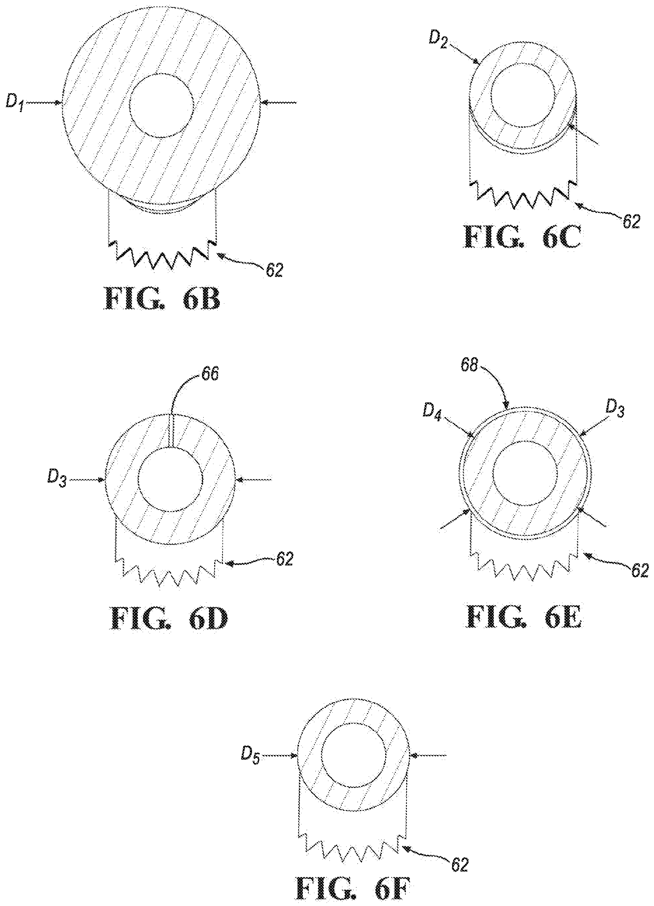

As shown in Figures 6B-6F, the shaft 48 may have a greater cross sectional area on the intermediate region 37 at location D1 than a cross sectional area at location D2. Figure 6B shows the cross sectional area of the shaft 48 at location D1. Figure 6C shows the cross sectional area of the shaft 48 at D2. Furthermore, Figure 6D shows the cross sectional area of the shaft 48 at location D3 and also shows the aperture 66. As shown in Figure 6E, the cross sectional area of D4 is less than the cross sectional area of D3. Figure 6F shows the cross sectional area at location D5. When taking figures 6B-6F together, this shows that the cross sectional area at location D3, which is near the protrusion 68, is greater than the cross sectional area at locations D4 and D5 which are proximally and distally adjacent to the protrusion, respectfully. The various cross-sectional area of the shaft 48 results in different strengths of the shaft 48 at various locations along the length. Generally, the greater cross-sectional area of the shaft 48 at a given location results in a greater wall thickness, which results in a greater strength.

As described above, the aperture 66 weakens the strength of the shaft 48. The greater cross sectional area at the protrusion 68 overcomes the weakness created by the aperture 66 to allow the ultrasonic tip 26 to be used at a higher power setting and not break from the increased stress. The cross sectional area of the protrusion 68 may be configured to have the least amount of area necessary to avoid inefficiencies and maintain line of sight, while maintaining necessary stress properties to prevent failure.

As shown in Figure 8, the shaft 48 of the ultrasonic tip 26 has varying thicknesses. Assuming the aspiration lumen 52 has a constant diameter from the distal region 50, through the intermediate region 37, to the proximal region 36, the thickness of shaft 48 at the aperture 66 is greater than the thickness of the shaft 48 at areas immediately adjacent to the aperture. Said another way, the shaft 48 thickness at the median region 80 is greater than the shaft 48 thickness at the first taper region 76 and the second taper region 78. The increased thickness of the shaft 48 at the median region 80 where the aperture 66 is located may improve the strength of the shaft 48.

As shown in Figure 4, the irrigation sleeve 24 is configured to surround a portion of the shaft 48 in when the irrigation sleeve 24 and the ultrasonic tip 26 are coupled to the handpiece 11. The irrigation sleeve 24 comprises the lumen 70. The irrigation sleeve 24 has a proximal end 36 and the distal end 94. The proximal end 36 of the sleeve 24 includes the coupling mechanism 38 configured to removeably couple to the handpiece 11. The irrigation sleeve defines an irrigation channel 88 and is separate from the lumen 70. The irrigation channel 88 has a distal end and a proximal end, the proximal end of the irrigation channel 88 is configured to releasably couple to the irrigation source (not shown). The irrigation channel 88 may carry fluid to the lumen 70 through an irrigation port 92. The irrigation port 92 may be in fluid communication with the irrigation channel 88 and the lumen 70.

In certain configurations, the fluid may flow from the irrigation source (not shown) through the irrigation channel 88 to the irrigation port 92. The irrigation port 92 supplies fluid to the lumen 70 of the irrigation sleeve 24. Fluid in the lumen 70 of the irrigation sleeve 24 may flow distally towards the cutting tip 26. While the fluid is flowing toward the cutting tip 26 some fluid may enter the aspiration lumen 52 through the aperture 66 on the protrusion 68. The fluid flowing through the aperture 66 helps to reduce the temperature of the cutting tip 26 and may increase performance of the surgical handpiece assembly 10. Furthermore, it may alternatively help reduce the temperature of the sleeve 24, to thereby prevent tissue that comes into contact with the exterior surface of the sleeve from being inadvertently heated.

The irrigation sleeve 24 may be made from any polymer, for example a thermoplastic. The distal end 94 of the irrigation sleeve 24 may have a portion of frangible sections that could be cut or snipped to change the length of the irrigation sleeve 24.

The irrigation channel 88 may be in fluid communication with the handpiece 11 and/or an irrigation source (not shown). The combination of the aperture 66 on the shaft 48, the irrigation channel 88, the position of the distal end 94 of the sleeve 24 relative to the aperture 66, the irrigation port 92 and the aspiration lumen 52 improve the cooling ability of the ultrasonic tip 26. Additionally, the above referenced combination improves cooling of the ultrasonic tip 26 to prevent the irrigation sleeve 24 from becoming deformed or melting from excessive heat generated by the longitudinal and torsional motion of the ultrasonic tip 26.

As shown in Figure 9, the irrigation sleeve 24 surrounds a portion of the ultrasonic tip 26 along the length of the ultrasonic tip. The distal end 94 of the irrigation sleeve 24 extends along the longitudinal axis 46 towards the distal region 50 of the ultrasonic tip 26. The distal end 94 of the irrigation sleeve 24 may extend beyond the aperture 66. The aperture 66 may have a proximal end and a distal end, the distal end 94 of the irrigation sleeve 24 extends beyond both the proximal side and the distal side of the aperture 66. In other words, the aperture 66 is completely covered by the sleeve 24 when the ultrasonic tip and the sleeve 24 are coupled to the handpiece 11. The fluid in the lumen 70 helps cool the ultrasonic tip 26 and the sleeve 24.

The ultrasonic tip 26 allows the efficient removal of bone with torsional or longitudinal motion of the instrument tip. The cutting feature 62 may comprise a cutting face 64A to aid in such removal. However, it should be appreciated that the ultrasonic tip 26 may also be used with transducers that vibrate longitudinally, torsionally, or a combination of both longitudinal and torsional motion. Furthermore, in some examples, the ultrasonic tip is free from the vibration conversion mechanism.

A torsional dissection tip is effective in the removal of bone, bony prominences, calcified neoplasm, cartilage, cartilaginous materials, intervertebral disc, and other pathologies when the cutting feature contacts such. The device is especially useful during neurosurgery, especially inside-out bone dissection once the superficial cortex has been removed, spinal surgery, orthopedic surgery, plastic/reconstructive surgery, and ear, nose, throat surgery, and other surgeries whereby the aforesaid tissues are encountered.

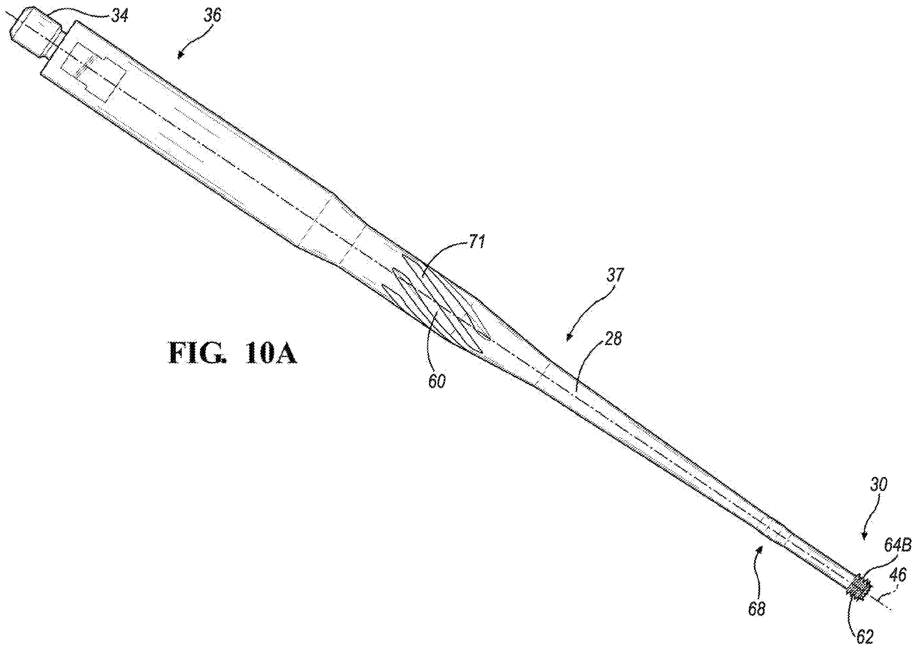

It is to be appreciated that the cutting features 62 of the ultrasonic tip 26 may have a plurality of configurations. Referring to Figures 10A-10E, various exemplary configurations of the cutting feature 62 of the ultrasonic tip 26 are illustrated. The cutting feature 62 may comprise a cutting face 64, wherein the cutting face 64 comprises one or more teeth 65. The teeth 65 of the cutting face 64 may be arranged wherein the teeth 65 are directed radially outward from the longitudinal axis 46 of the ultrasonic tip 26. For certain configurations, the plane of the cutting feature is substantially parallel yet offset to the central axis of the distal end 50 of the instrument, however the position of the cutting face 64 can be varied in a virtually limitless manner.

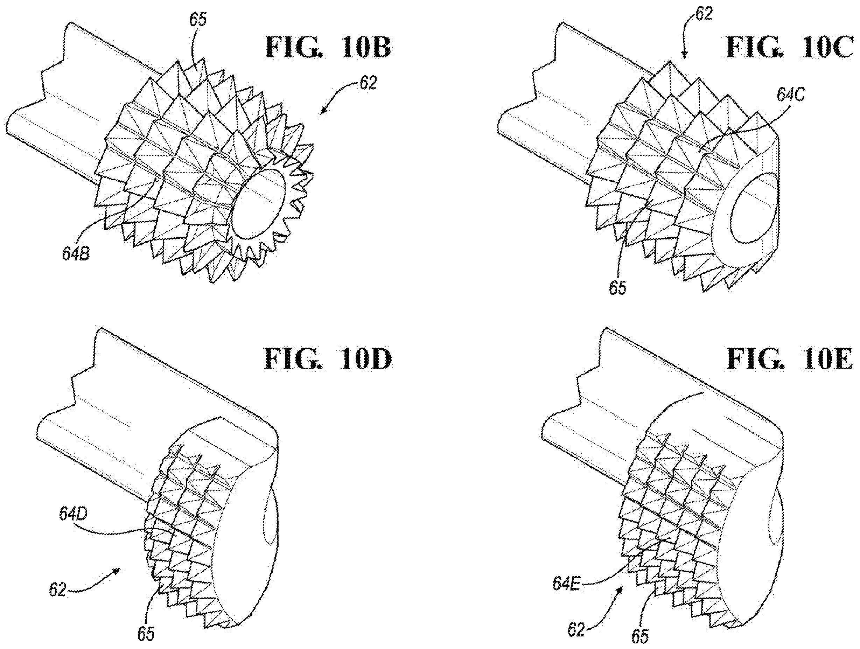

Referring to Figure 10B, a first configuration of the cutting feature 62 is illustrated. The first configuration of the cutting feature 62 comprises a cutting surface 64B that includes a plurality of teeth 65 disposed radially about the distal end 50 of the shaft 48. For example, the teeth 65 may configured to be directed outwardly from the longitudinal axis 46 of the ultrasonic tip 26, and the teeth 65 arrange to encircle the entire circumference of the distal end of the shaft 48. At least some of the teeth extend distally.

Alternatively, Figure 10C illustrates a second configuration of the cutting feature 62. The second configuration of the cutting feature 62 comprises a cutting surface 64C comprising an arched or half cylinder-like shape that includes a plurality of teeth 65 directed outwardly from the longitudinal axis of the ultrasonic tip 26.

Figure 10D illustrates a third configuration of the cutting feature 62. The third configuration of the cutting feature 62 comprises a cutting surface 64D comprising an arched or half cylinder-like shape that includes a plurality of teeth 65 directed outwardly from the longitudinal axis of the ultrasonic tip 26. The cutting surface 64D is generally offset from the shaft 48 of the ultrasonic tip 26 and oriented to be perpendicular to the longitudinal axis 46 of the ultrasonic tip 26.

Figure 10E illustrates a fourth configuration of the cutting feature 62. The fourth configuration of the cutting feature 62 comprises a cutting surface 64E comprising an arched or half cylinder-like shape that includes a plurality of teeth 65 directed outwardly from the longitudinal axis of the ultrasonic tip 26. The cutting surface 64E is generally offset from the shaft of the ultrasonic tip 26 and oriented to be perpendicular to the longitudinal axis 46 of the ultrasonic tip 26. Other suitable cutting features are also contemplated, including those described in U.S. Patent No. 8,512,340; and U.S. Publication 2018/0103976.

Several examples have been discussed in the foregoing description. However, the examples discussed herein are not intended to be exhaustive or limit the invention to any particular form. For example, while the example configurations describe the surgical instrument as an ultrasonic handpiece, it is further contemplated that the features and concepts described with regard to the ultrasonic handpiece may be applied to other medical or surgical instruments. This similarly applies to the ultrasonic tip, which may further include blades, drill bits, rotating burs, open-window shavers, and the like. The terminology which has been used is intended to be in the nature of words of description rather than of limitation. The invention is defined by the appended claims.

## Claims

1. An ultrasonic sleeve assembly (14) for use with a surgical handpiece (11) comprising a transducer (32) disposed in a housing, the ultrasonic sleeve assembly (14) comprising:
a cutting tip (26) comprising:
a shaft (48) comprising a distal end (50), and a proximal end (36), wherein said proximal end (36) comprises a coupling feature (34) configured to removeably couple said shaft (48) to said handpiece (11);
a cutting feature (62) coupled to said distal end (50) of said shaft (48);
an aspiration lumen (52) defined by said shaft (48), said aspiration lumen (52) configured to extend along a longitudinal axis (46) of said shaft (48), said aspiration lumen (52) in fluid communication with said handpiece (11); and
an aperture (66) in fluid communication with said aspiration lumen (52), located between said proximal end (36) and said distal end (50) of said shaft (48), the aperture (66) having a proximal end and a distal end; and
an irrigation sleeve (24) configured to surround a portion of said shaft (48) when said irrigation sleeve (24) and said cutting tip (26) are coupled to said handpiece (11), said irrigation sleeve (24) having a proximal end and a distal end (94), wherein said irrigation sleeve (24) defines a lumen (70), said proximal end of said irrigation sleeve (24) having a coupling mechanism (38) configured to removeably couple said irrigation sleeve (24) to said handpiece (11), wherein said irrigation sleeve (24) further defines an irrigation channel (88) comprising a distal end and a proximal end, said proximal end of said irrigation channel (88) being configured to receive irrigation fluid from an irrigation source, and wherein an irrigation port (92) is positioned adjacent the distal end (94) of the sleeve (24) and in fluid communication with said distal end of said irrigation channel (88), said irrigation port (92) configured to dispense irrigation fluid toward said shaft (48),
wherein said irrigation sleeve (24) and said cutting tip (26) are configured such that when said irrigation sleeve (24) and cutting tip (26) are coupled to said handpiece (11):
said distal end (94) of said irrigation sleeve (24) is positioned proximal to said distal end (50) of said shaft (48);
said irrigation port (92) is positioned proximal relative to said aperture (66) along said longitudinal axis (46); and
said distal end (94) of the irrigation sleeve (24) is distal to said distal end of said aperture (66) such that said irrigation sleeve (24) encompasses an entirety of said aperture (66).

2. The ultrasonic sleeve assembly (14) of claim 1, wherein said distal end (94) of said irrigation sleeve (24) is configured to extend beyond said distal end of said aperture (66) in a distal direction along said longitudinal axis (46) when said irrigation sleeve (24) and said cutting tip (26) are coupled to said handpiece (11).

3. The ultrasonic sleeve assembly (14) of claim 1 or 2, wherein said coupling feature (34) comprises a plurality of threads configured to couple to said transducer (32) of said handpiece (11).

4. The ultrasonic sleeve assembly (14) of any one of claims 1 to 3, wherein said shaft (48) comprises a vibration conversion mechanism (60) for converting a vibration energy transmitted from said transducer (32) into a composite vibration composed of a longitudinal vibration along the said longitudinal axis (46) and a torsional vibration.

5. The ultrasonic sleeve assembly (14) of claim 4, wherein said vibration conversion mechanism (60) comprising one or more helical groove portions (71) formed on an external surface of said shaft (48).

6. The ultrasonic sleeve assembly (14) of claim 4 or 5, wherein said aperture (66) is positioned on said shaft (48) distal to said vibration conversion mechanism (60).

7. The ultrasonic sleeve assembly (14) of claim 6, wherein said shaft (48) comprises a distal region defining said shaft distal end (50), a proximal region defining said shaft proximal end (36), and an intermediation region (37), and wherein said aperture (66) is positioned between said shaft distal region and said shaft intermediate region, and said intermediate region (37) is positioned distal to said vibration conversion mechanism (60).

8. The ultrasonic sleeve assembly (14) of any one of any one of claims 1 to 7, wherein said cutting feature (62) comprises a cutting face (64, 64A) facing in a direction perpendicular to said longitudinal axis (46).

9. The ultrasonic sleeve assembly (14) of claim 8, wherein said aperture (66) is positioned in a radially opposed direction from said cutting face (64, 64A).

10. The ultrasonic sleeve assembly (14) of any one of claims 1 to 7, wherein said cutting feature (62) has a cutting face (64, 64B, 64C, 64D, 64E) arranged radially outward from said longitudinal axis (46) and wherein said cutting face (64, 64B, 64C, 64D, 64E) comprises one or more teeth (65).

11. The ultrasonic sleeve assembly (14) of any one of claims 1 to 10, wherein said aspiration lumen (52) is open at said distal end (50) and said proximal end (36) of said shaft (48).

12. The ultrasonic sleeve assembly (14) of any one of claims 1 to 11, comprising a protrusion (68) on said shaft (48) and positioned between said distal end (50) and said proximal end (36) of said shaft (48), a portion of said protrusion (68) having a first diameter (D1), wherein said first diameter (D1) is less than a second diameter (D2) of said proximal end (36) of said shaft (48) and greater than a third diameter (D3) of said distal end (50) of said shaft (48), and wherein said protrusion (68) defines said aperture (66) and reinforces an area surrounding said aperture (66).

13. The ultrasonic sleeve assembly (14) of any one of claims 1 to 11, comprising a protrusion (68) on said shaft and positioned between said distal end (50) and said proximal end (36) of said shaft (48), a portion of said protrusion (68) having a first cross-sectional area, wherein said first cross-sectional area is less than a second cross-sectional area of said proximal end (36) of said shaft (48) and greater than a third cross-sectional area of said distal end (50) of said shaft (48), and wherein said protrusion (68) defines said aperture (66) and reinforces an area surrounding said aperture (66).

14. The ultrasonic sleeve assembly (14) of claim 12 or 13, wherein said protrusion (68) comprises a first taper region (76), a median region (80), and a second taper region (78), with an external surface of said median region (80) being generally parallel to the longitudinal axis (46) and said median region (80) disposed between said first taper region (76) and said second taper region (78), said first taper region (76) being proximal to said median region (80) and having a positive slope, and said second taper region (78) being distal to said median region (80) and having a negative slope.

15. The ultrasonic sleeve assembly (14) of any of claims 12 to 14, wherein an axis of said aperture (66) is transverse to said longitudinal axis (46).

## Patentansprüche

1. Ultraschallhülsenanordnung (14) zur Verwendung mit einem chirurgischen Handstück (11), das einen in einem Gehäuse angeordneten Wandler (32) umfasst, wobei die Ultraschallhülsenanordnung (14) umfasst:
eine Schneidspitze (26), umfassend:
einen Schaft (48), der ein distales Ende (50) und ein proximales Ende (36) umfasst, wobei das proximale Ende (36) ein Kopplungsmerkmal (34) umfasst, das dazu ausgebildet ist, den Schaft (48) lösbar mit dem Handstück (11) zu koppeln;
ein Schneidmerkmal (62), das mit dem distalen Ende (50) des Schafts (48) gekoppelt ist;
ein durch den Schaft (48) definiertes Ansauglumen (52), wobei das Ansauglumen (52) dazu ausgebildet ist, sich entlang einer Längsachse (46) des Schafts (48) zu erstrecken, und wobei das Ansauglumen (52) in Fluidverbindung mit dem Handstück (11) steht; sowie
eine Öffnung (66), die in Fluidverbindung mit dem Ansauglumen (52) steht und sich zwischen dem proximalen Ende (36) und dem distalen Ende (50) des Schafts (48) befindet, wobei die Öffnung (66) ein proximales Ende und ein distales Ende aufweist; und
eine Spülhülse (24), die dazu ausgebildet ist, einen Teil des Schafts (48) zu umgeben, wenn die Spülhülse (24) und die Schneidspitze (26) mit dem Handstück (11) gekoppelt sind, wobei die Spülhülse (24) ein proximales Ende und ein distales Ende (94) aufweist, wobei die Spülhülse (24) ein Lumen (70) definiert und das proximale Ende der Spülhülse (24) einen Kopplungsmechanismus (38) aufweist, der dazu ausgebildet ist, die Spülhülse (24) lösbar mit dem Handstück (11) zu koppeln, wobei die Spülhülse (24) ferner einen Spülkanal (88) definiert, der ein distales Ende und ein proximales Ende umfasst, wobei das proximale Ende des Spülkanals (88) dazu ausgebildet ist, Spülflüssigkeit aus einer Spülquelle zu erhalten, und wobei ein Spülanschluss (92) benachbart zum distalen Ende (94) der Hülse (24) ist und in Fluidverbindung mit dem distalen Ende des Spülkanals (88) steht, wobei der Spülanschluss (92) dazu ausgebildet ist, Spülflüssigkeit in Richtung des Schafts (48) abzugeben,
wobei die Spülhülse (24) und die Schneidspitze (26) dazu ausgebildet sind, dass, wenn die Spülhülse (24) und die Schneidspitze (26) mit dem Handstück (11) gekoppelt sind:
das distale Ende (94) der Spülhülse (24) proximal zum distalen Ende (50) des Schafts (48) angeordnet ist;
der Spülanschluss (92) entlang der Längsachse (46) proximal bezüglich der Öffnung (66) positioniert ist; und
das distale Ende (94) der Spülhülse (24) distal zum distalen Ende der Öffnung (66) liegt, sodass die Spülhülse (24) die gesamte Öffnung (66) umgibt.

2. Ultraschallhülsenanordnung (14) nach Anspruch 1, wobei das distale Ende (94) der Spülhülse (24) dazu ausgebildet ist, sich in distaler Richtung entlang der Längsachse (46) über das distale Ende der Öffnung (66) hinaus zu erstrecken, wenn die Spülhülse (24) und die Schneidspitze (26) mit dem Handstück (11) gekoppelt sind.

3. Ultraschallhülsenanordnung (14) nach Anspruch 1 oder 2, wobei das Kopplungsmerkmal (34) eine Mehrzahl von Gewindegängen umfasst, die dazu ausgebildet sind, mit dem Wandler (32) des Handstücks (11) gekoppelt zu werden.

4. Ultraschallhülsenanordnung (14) nach einem der Ansprüche 1 bis 3, wobei der Schaft (48) einen Schwingungsumwandlungsmechanismus (60) umfasst, um eine vom Wandler (32) übertragene Schwingungsenergie in eine zusammengesetzte Schwingung umzuwandeln, die sich aus einer Längsschwingung entlang der Längsachse (46) und einer Torsionsschwingung zusammensetzt.

5. Ultraschallhülsenanordnung (14) nach Anspruch 4, wobei der Schwingungsumwandlungsmechanismus (60) einen oder mehrere spiralförmige Rillenabschnitte (71) umfasst, die an einer Außenfläche des Schafts (48) ausgebildet sind.

6. Ultraschallhülsenanordnung (14) nach Anspruch 4 oder 5, wobei die Öffnung (66) distal zum Schwingungsumwandlungsmechanismus (60) an dem Schaft (48) positioniert ist.

7. Ultraschallhülsenanordnung (14) nach Anspruch 6, wobei der Schaft (48) einen distalen Bereich, der das distale Schaftende (50) definiert, einen proximalen Bereich, der das proximale Schaftende (36) definiert, sowie einen Zwischenbereich (37) umfasst, und wobei die Öffnung (66) zwischen dem distalen Schaftbereich und dem Zwischenbereich des Schafts positioniert ist und der Zwischenbereich (37) distal zum Schwingungsumwandlungsmechanismus (60) positioniert ist.

8. Ultraschallhülsenanordnung (14) nach einem der Ansprüche 1 bis 7, wobei das Schneidmerkmal (62) eine Schneidfläche (64, 64A) umfasst, die in eine Richtung senkrecht zur Längsachse (46) weist.

9. Ultraschallhülsenanordnung (14) nach Anspruch 8, wobei die Öffnung (66) in einer radial entgegengesetzten Richtung zur Schneidfläche (64, 64A) positioniert ist.

10. Ultraschallhülsenanordnung (14) nach einem der Ansprüche 1 bis 7, wobei das Schneidmerkmal (62) eine Schneidfläche (64, 64B, 64C, 64D, 64E) aufweist, die von der Längsachse (46) radial nach außen angeordnet ist, und wobei die Schneidfläche (64, 64B, 64C, 64D, 64E) einen oder mehrere Zähne (65) umfasst.

11. Ultraschallhülsenanordnung (14) nach einem der Ansprüche 1 bis 10, wobei das Ansauglumen (52) am distalen Ende (50) und am proximalen Ende (36) des Schafts (48) offen ist.

12. Ultraschallhülsenanordnung (14) nach einem der Ansprüche 1 bis 11, umfassend einen Vorsprung (68) an dem Schaft (48), der zwischen dem distalen Ende (50) und dem proximalen Ende (36) des Schafts (48) positioniert ist, wobei ein Teil des Vorsprungs (68) einen ersten Durchmesser (D1) aufweist, wobei der erste Durchmesser (D1) kleiner ist als ein zweiter Durchmesser (D2) des proximalen Endes (36) des Schafts (48) und größer als ein dritter Durchmesser (D3) des distalen Endes (50) des Schafts (48), und wobei der Vorsprung (68) die Öffnung (66) definiert und einen die Öffnung (66) umgebenden Bereich verstärkt.

13. Ultraschallhülsenanordnung (14) nach einem der Ansprüche 1 bis 11, umfassend einen Vorsprung (68) an dem Schaft, der zwischen dem distalen Ende (50) und dem proximalen Ende (36) des Schafts (48) positioniert ist, wobei ein Teil des Vorsprungs (68) eine erste Querschnittsfläche aufweist, wobei die erste Querschnittsfläche kleiner ist als eine zweite Querschnittsfläche des proximalen Endes (36) des Schafts (48) und größer als eine dritte Querschnittsfläche des distalen Endes (50) des Schafts (48), und wobei der Vorsprung (68) die Öffnung (66) definiert und einen die Öffnung (66) umgebenden Bereich verstärkt.

14. Ultraschallhülsenanordnung (14) nach Anspruch 12 oder 13, wobei der Vorsprung (68) einen ersten Verjüngungsbereich (76), einen mittleren Bereich (80) und einen zweiten Verjüngungsbereich (78) umfasst, wobei eine Außenfläche des mittleren Bereichs (80) im Wesentlichen parallel zur Längsachse (46) verläuft und der mittlere Bereich (80) zwischen dem ersten Verjüngungsbereich (76) und dem zweiten Verjüngungsbereich (78) angeordnet ist, und wobei der erste Verjüngungsbereich (76) proximal zum mittleren Bereich (80) liegt und eine positive Steigung aufweist und der zweite Verjüngungsbereich (78) distal zum mittleren Bereich (80) liegt und eine negative Steigung aufweist.

15. Ultraschallhülsenanordnung (14) nach einem der Ansprüche 12 bis 14, wobei eine Achse der Öffnung (66) quer zur Längsachse (46) verläuft.

## Revendications

1. Ensemble manchon à ultrasons (14) destiné à être utilisé avec une pièce à main chirurgicale (11) comprenant un transducteur (32) disposé dans un boîtier, l'ensemble manchon à ultrasons (14) comprenant:
un embout de coupe (26) comprenant:
une tige (48) comportant une extrémité distale (50) et une extrémité proximale (36), ladite extrémité proximale (36) comprenant un élément d'accouplement (34) conçu pour accoupler de manière amovible ladite tige (48) à ladite pièce à main (11);
un élément de coupe (62) relié à ladite extrémité distale (50) de ladite tige (48);
une lumière d'aspiration (52) délimitée par ladite tige (48), ladite lumière d'aspiration (52) étant conçue pour s'étendre le long d'un axe longitudinal (46) de ladite tige (48), ladite lumière d'aspiration (52) étant en communication fluidique avec ladite pièce à main (11); et
une ouverture (66) en communication fluidique avec ladite lumière d'aspiration (52), située entre ladite extrémité proximale (36) et ladite extrémité distale (50) de ladite tige (48), l'ouverture (66) comportant une extrémité proximale et une extrémité distale; et
un manchon d'irrigation (24) conçu pour entourer une partie de ladite tige (48) lorsque ledit manchon d'irrigation (24) et ledit embout de coupe (26) sont reliés à ladite pièce à main (11), ledit manchon d'irrigation (24) comportant une extrémité proximale et une extrémité distale (94), dans lequel ledit manchon d'irrigation (24) délimite une lumière (70), ladite extrémité proximale dudit manchon d'irrigation (24) comportant un mécanisme d'accouplement (38) conçu pour accoupler de manière amovible ledit manchon d'irrigation (24) à ladite pièce à main (11), ledit manchon d'irrigation (24) délimitant en outre un canal d'irrigation (88) comprenant une extrémité distale et une extrémité proximale, ladite extrémité proximale dudit canal d'irrigation (88) étant conçue pour recevoir un fluide d'irrigation provenant d'une source d'irrigation, et un orifice d'irrigation (92) étant positionné à proximité de l'extrémité distale (94) du manchon (24) et en communication fluidique avec ladite extrémité distale dudit canal d'irrigation (88), ledit orifice d'irrigation (92) étant conçu pour distribuer du fluide d'irrigation vers ladite tige (48),
dans lequel ledit manchon d'irrigation (24) et ledit embout de coupe (26) sont conçus de telle sorte que, lorsque ledit manchon d'irrigation (24) et ledit embout de coupe (26) sont reliés à ladite pièce à main (11):
ladite extrémité distale (94) dudit manchon d'irrigation (24) est positionnée proximalement par rapport à ladite extrémité distale (50) de ladite tige (48);
ledit orifice d'irrigation (92) est positionné proximalement à ladite ouverture (66) le long dudit axe longitudinal (46); et
ladite extrémité distale (94) du manchon d'irrigation (24) est distale par rapport à ladite extrémité distale de ladite ouverture (66), de telle sorte que ledit manchon d'irrigation (24) englobe une intégralité de ladite ouverture (66).

2. Ensemble manchon à ultrasons (14) selon la revendication 1, dans lequel ladite extrémité distale (94) dudit manchon d'irrigation (24) est conçue pour s'étendre au-delà de ladite extrémité distale de ladite ouverture (66) dans une direction distale le long dudit axe longitudinal (46) lorsque ledit manchon d'irrigation (24) et ladite pointe de coupe (26) sont reliés à ladite pièce à main (11).

3. Ensemble manchon à ultrasons (14) selon la revendication 1 ou 2, dans lequel ledit élément d'accouplement (34) comprend une pluralité de filets conçus pour se raccorder audit transducteur (32) de ladite pièce à main (11).

4. Ensemble manchon à ultrasons (14) selon l'une quelconque des revendications 1 à 3, dans lequel ladite tige (48) comprend un mécanisme de conversion de vibrations (60) pour convertir une énergie vibratoire transmise par ledit transducteur (32) en une vibration composite constituée d'une vibration longitudinale le long dudit axe longitudinal (46) et d'une vibration torsionnelle.

5. Ensemble manchon à ultrasons (14) selon la revendication 4, dans lequel ledit mécanisme de conversion de vibrations (60) comprend une ou plusieurs portions de rainure hélicoïdale (71) formées sur une surface externe de ladite tige (48).

6. Ensemble manchon à ultrasons (14) selon la revendication 4 ou 5, dans lequel ladite ouverture (66) est située sur ladite tige (48) distalement par rapport audit mécanisme de conversion de vibrations (60).

7. Ensemble manchon à ultrasons (14) selon la revendication 6, dans lequel ladite tige (48) comprend une région distale délimitant ladite extrémité distale de tige (50), une région proximale délimitant ladite extrémité proximale (36) de l'arbre, et une région intermédiaire (37), et dans lequel ladite ouverture (66) est positionnée entre ladite région distale de l'arbre et ladite région intermédiaire de tige, et ladite région intermédiaire (37) est positionné distalement par rapport audit mécanisme de conversion de vibrations (60).

8. Ensemble manchon à ultrasons (14) selon l'une quelconque des revendications 1 à 7, dans lequel ledit élément de coupe (62) comprend une face de coupe (64, 64A) orientée dans une direction perpendiculaire audit axe longitudinal (46).

9. Ensemble manchon à ultrasons (14) selon la revendication 8, dans lequel ladite ouverture (66) est positionnée dans une direction radialement opposée à ladite face de coupe (64, 64A).

10. Ensemble manchon à ultrasons (14) selon l'une quelconque des revendications 1 à 7, dans lequel ledit élément de coupe (62) comporte une face de coupe (64, 64B, 64C, 64D, 64E) disposée radialement vers l'extérieur par rapport audit axe longitudinal (46) et dans lequel ladite face de coupe (64, 64B, 64C, 64D, 64E) comprend une ou plusieurs dents (65).

11. Ensemble manchon à ultrasons (14) selon l'une quelconque des revendications 1 à 10, dans lequel ladite lumière d'aspiration (52) est ouverte au niveau de ladite extrémité distale (50) et de ladite extrémité proximale (36) de ladite tige (48).

12. Ensemble manchon à ultrasons (14) selon l'une quelconque des revendications 1 à 11, comprenant une saillie (68) sur ladite tige (48) et positionnée entre ladite extrémité distale (50) et ladite extrémité proximale (36) de ladite tige (48), une partie de ladite saillie (68) présentant un premier diamètre (D1), ledit premier diamètre (D1) étant inférieur à un deuxième diamètre (D2) de ladite extrémité proximale (36) de ladite tige (48) et supérieur à un troisième diamètre (D3) de ladite extrémité distale (50) de ladite tige (48), et ladite saillie (68) délimitant ladite ouverture (66) et renforce une zone entourant ladite ouverture (66).

13. Ensemble manchon à ultrasons (14) selon l'une quelconque des revendications 1 à 11, comprenant une saillie (68) sur ladite tige et positionnée entre ladite extrémité distale (50) et ladite extrémité proximale (36) de ladite tige (48), une partie de ladite saillie (68) présentant une première section transversale, ladite première section transversale étant inférieure à une deuxième section transversale de ladite extrémité proximale (36) de ladite tige (48) et supérieure à une troisième section transversale de ladite extrémité distale (50) de ladite tige (48), et ladite saillie (68) délimitant ladite ouverture (66) et renforce une zone entourant ladite ouverture (66).

14. Ensemble manchon à ultrasons (14) selon la revendication 12 ou 13, dans lequel ladite saillie (68) comprend une première zone conique (76), une zone médiane (80), et une deuxième zone conique (78), la surface externe de ladite zone médiane (80) étant globalement parallèle à l'axe longitudinal (46) et ladite zone médiane (80) étant disposée entre ladite première zone conique (76) et ladite deuxième zone conique (78), ladite première zone conique (76) étant proximale par rapport à ladite zone médiane (80) et présentant une pente positive, et ladite seconde zone conique (78) étant distale par rapport à ladite zone médiane (80) et présentant une pente négative.

15. Ensemble manchon à ultrasons (14) selon l'une quelconque des revendications 12 à 14, dans lequel un axe de ladite ouverture (66) est transversal audit axe longitudinal (46).
